(19) Europäisches Patentamt
European Patent Office
Office européen des brevets

(11) Publication number: **0 239 122 B1**

# (12) EUROPEAN PATENT SPECIFICATION

(45) Date of publication of patent specification: **17.06.92**

(51) Int. Cl.[5]: **C12P 41/00**, C12P 13/02

(21) Application number: **87104586.0**

(22) Date of filing: **27.03.87**

## (54) Process for the enzymatic resolution of racemic 2-amino-1-alkanols.

(30) Priority: **28.03.86 IT 1994486**

(43) Date of publication of application:
**30.09.87 Bulletin 87/40**

(45) Publication of the grant of the patent:
**17.06.92 Bulletin 92/25**

(84) Designated Contracting States:
**AT BE CH DE ES FR GB LI NL SE**

(56) References cited:

**PATENT ABSTRACTS OF JAPAN, vol. 8, no. 126 (C-228)[1563], 13th June 1984; & JP-A-59 39 294 (HAMARI YAKUHIN KOGYO K.K.) 03-03-1984**

**PATENT ABSTRACTS OF JAPAN, vol. 8, no. 34 (C-210)[1471], 15th February 1984; & JP-A-58 198 296 (CHISSO K.K.) 18-11-1983**

**THE JOURNAL OF ORGANIC CHEMISTRY, vol. 52, no. 23, 13th November 1987, pages 5079-5082, American Chemical Society; F. FRANCALANCI et al.: "Lipase-catalyzed resolution of chiral 2-amino 1-alcohols"**

(73) Proprietor: **Montedison S.p.A.**
**31, Foro Buonaparte**
**I-20121 Milan(IT)**

(72) Inventor: **Francalanci, Franco**
**7, Via G. Ferraris**
**I-28100 Novara(IT)**
Inventor: **Cesti, Pietro**
**51, Via Torino**
**I-28069 Trecate Novara(IT)**
Inventor: **Foa', Marco**
**19, Via Del Sabbione**
**I-28100 Novara(IT)**
Inventor: **Martinengo, Tiziano**
**34, Via Carlo Alberto**
**I-28064 Carpignano Sesia Novara(IT)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40(DE)**

EP 0 239 122 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the enzymatic resolution of the optical isomers of racemic 2-amino-1-alkanols having general formula (I):

(R,S) 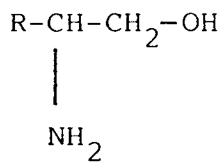 (I)

wherein R represents a $C_1$-$C_8$ alkyl group.

The optically active 2-amino-1-alkanol compounds obtained represent an important class of intermediates which may be used in the synthesis of biologically active products. For instance, 2-amino-1-butanol in its S(+) form is a useful intermediate for the synthesis of an important antituberculosis agent, i.e., (+) 2,2'-(ethylenediimino)-bis-[I-butanol], (ETHAMBUTOL).

Some processes are known for the separation of the optical antipodes of racemic (S,R) 2-amino-1-butanol through formation of diastereoisomeric salts by means of optically active acids US-A-3 944 608; EP-A 36 265; Hung Teljes 19 369).

The known methods for the resolution of the two optical isomers, however, are complicated, need optically active reactants that are expensive, require a careful control of the reaction conditions and involve several crystallization steps of the products; therefore they are disadvantageous from an industrial point of view.

Recently a biological method has been described (JP-A-59-39 294) which is capable of resolving the (R, S) N-acetyl-2-amino-1-butanol compound into its optical isomers. Said method, however, does not seem to ensure sufficient advantages, especially from the industrial point of view, because of the complicated and expensive operations of regio-selective acylation that have to be carried out on the racemic 2-amino-1-butanol and of the quantitative separation of the products.

Therefore, there was the need of having a method at disposal, which could be carried out industrially and allows the resolution of the optical isomers of the two 2-amino-1-alkanols having formula (I) as defined hereinbefore, according to a simple, efficient and economic operating method.

Therefore the object of the present invention is to provide a process for carrying out the separation or resolution of the optical isomers of racemic 2-amino-1-alkanols (I), according to a simple method, which, in particular, is free from the drawbacks that may be found in the prior art.

It has now been found that this object may be reached according to a biotechnological process of enzymatic asymmetric hydrolysis, carried out by using particular enzymes endowed with a selective activity, on suitable racemic esters of (R, S) N-alkoxycarbonyl derivatives of racemic 2-amino-1-alkanols (I) having general formula (II) as defined hereinafter.

In practice, use is made of enzymes, belonging to the lipase class, which give rise to the hydrolysis reaction, acting only on the (R) form of aforesaid racemic esters (II) in a stereoselective way (asymmetric hydrolysis). Afterwards, non-reacted ester (II) in its (S) form and the corresponding (R) form that has been hydrolyzed, namely converted selectively into the corresponding (R) alcohol having general formula (III) as defined hereinafter, which both are obtained at the end of the enzymatic hydrolysis, are separated and hydrolyzed singly, according to usual methods and conditions, thereby obtaining the corresponding (R) and (S) 2-amino-1-alkanols separately, in their optically pure form.

Therefore the object of the present invention, defined in a more explicit way, consists in a process for the biotechnological separation, carried out by means of enzymes, of optical (R) and (S) isomers of racemic 2-amino-1-alkanols having general formula (I):

(R,S) 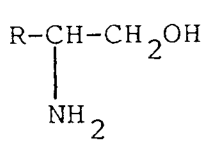 (I)

which process is characterized in that a racemic (R, S) N-alkoxycarbonyl ester derivative of said racemic 2-amino-1-alkanols (I) having general formula (II):

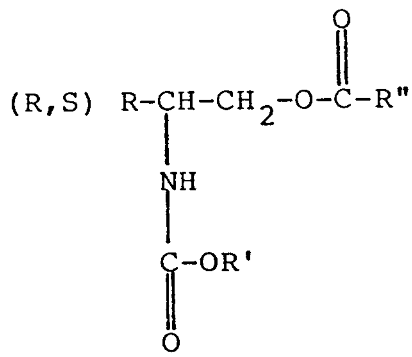

(II)

wherein R′ represents a $C_1$-$C_8$ alkyl group or a benzyl group and R and R″, which may be the same or different, represent $C_1$-$C_8$ alkyl groups, is reacted with a lipase which hydrolyzes, asymmetrically and in a prevalent way, the (R) form of the racemic ester having formula (II), and in that, afterwards, by operating substantially according to usual techniques, the obtained (R) alcohol, corresponding substantially to the (R) form of hydrolyzed racemic ester (II), is separated from non-reacted ester (II), substantially in its (S) form, which both are then hydrolyzed separately to give rise to (R) and (S) 2-amino-1-alkanol compounds (I) which are optically pure.

The starting compounds, i.e. the racemic esters having formula (II) are known compounds and/or may be synthesized according to usual techniques, under Schotten-Baumann's reaction conditions (see, for instance, Jerry March, Advanced Org. Chem. : Reactions Mechanisms and structure, Mac Graw-Hill Ed., page 382, 2nd edition, 1977). In practice, said method consists of a) reacting the racemic 2-amino-1-alkanols having formula (I) with alkyl-chloroformates having formula (IV), in an alkaline aqueous medium, according to the following reaction scheme:

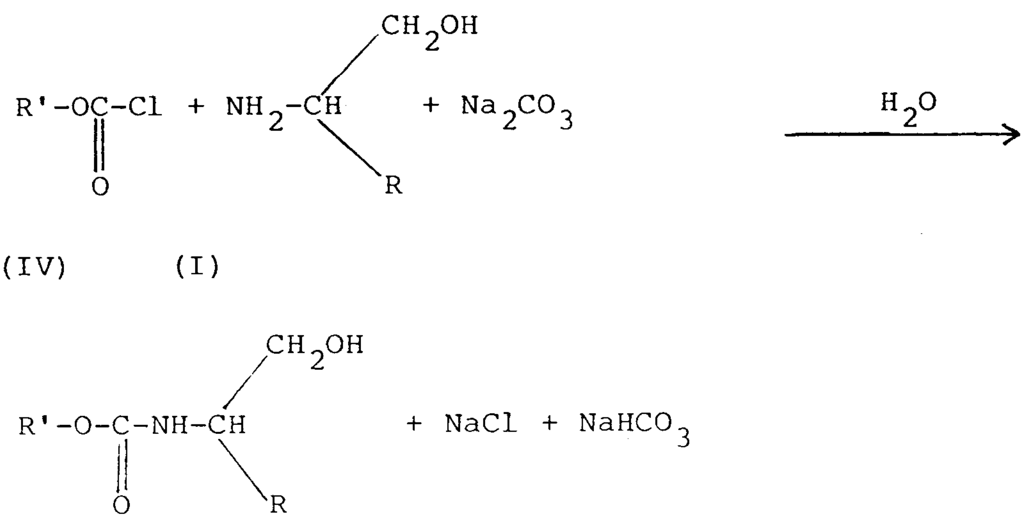


(IV) (I)

(III)

wherein R and R′ have the meanings defined hereinbefore; and b) afterwards esterifying the primary alcoholic group with an organic acid R"-COOH wherein R" has the aforesaid meanings, according to usual techniques.

According to a schematic description of the process of the present invention, the racemic esters having formula (II) are reacted with a lipase, in an aqueous solution, according to the following reaction scheme:

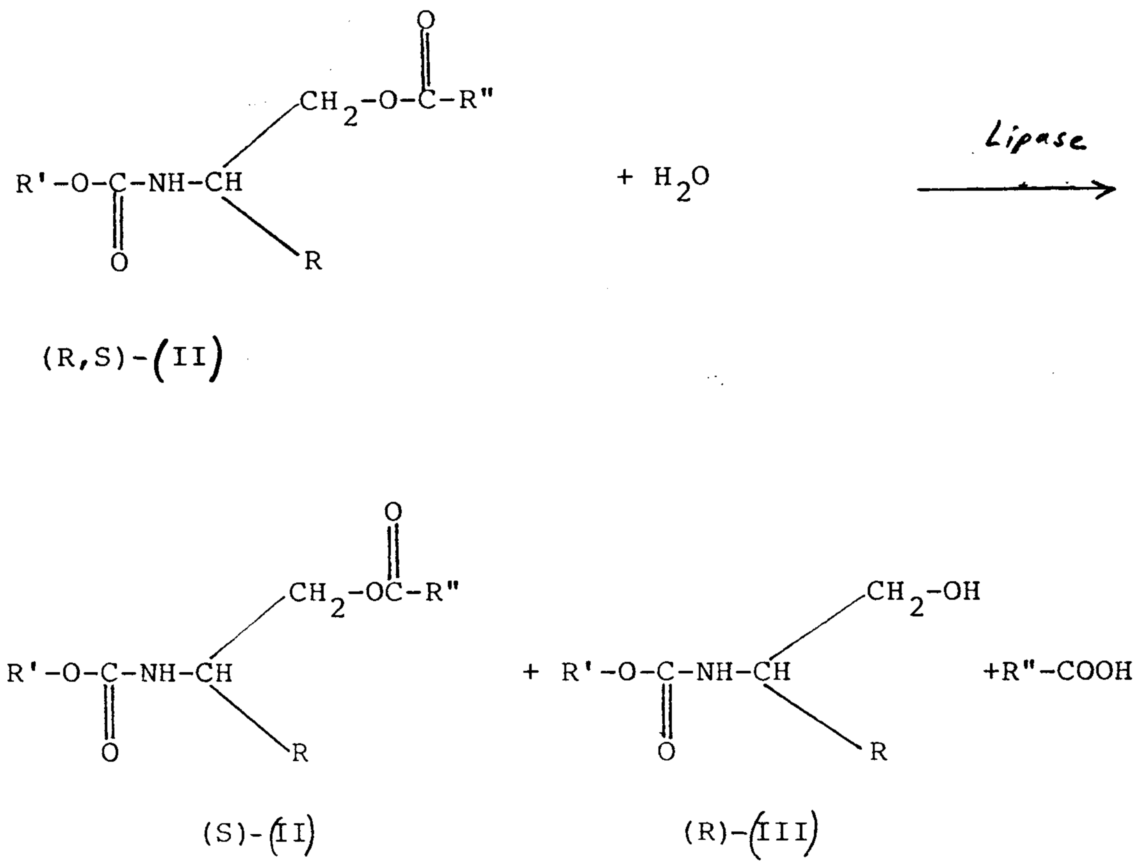


(R,S)-(II)

(S)-(II)

(R)-(III)

wherein R, R′ and R″ have the meanings defined hereinbefore.

According to the invention, a lipase is used as enzyme, said lipase being preferably selected from pancreatin and steapsin. Such lipases may be found on the market at low price and optionally may be used immobilized on inert substrates of different nature.

In the reaction use is made of weight ratios lipase: racemic ester having formula (II) ranging between about 1:1 and 1:200, preferably between 1:20 and 1:100.

The hydrolysis reaction of racemic esters (II) is carried out by stirring strongly the mixture consisting of the racemic ester having formula (II) with the lipase, in an aqueous solvent, at a temperature ranging between about 5° C and 60° C, preferably between 20° C and 40° C, keeping the pH value of the reaction mixture between about 5 and 9, preferably between 6 and 8.

The pH of the reaction medium is kept constant by using a sodium or potassium buffer solution or by neutralization of the forming acidity by means of a base, such as KOH, NaOH etc.

The concentration of starting racemic ester (II) in the reaction mixture may range between about 0.1 and 1 mole/litre, according to the substrate characteristics.

The reaction time of asymmetric hydrolysis may range between 2 and 40 hours, according to the selected working parameters.

When the reaction of asymmetric (stereoselective) hydrolysis is over, one starts the separation of the formed products; for example, the alcohol having formula (III), substantially rich in the (R) form and non-reacted ester having formula (II), rich in (S) isomer, may be extracted from the reaction mixture by using solvents immiscible with water, such as methylene chloride, chloroform, ether etc. Then the compounds (III) and (S) (II) are separated according to usual techniques such as, for instance, column chromatography, fractional distillation etc.

Alternatively and in a more advantageous way, the two products may be separate by exploiting their different solubility in water or by using suitable organic solvents immiscible with $H_2O$ such as benzene, toluene, hexane, etc.; usual techniques are involved.

Afterwards, in the second step of the process, of the present invention, a) the (S)-N-alkoxycarbonyl ester derivative of 2-amino-1-alkanol (I) having formula (II), which was not substantially involved in the asymmetric enzymatic hydrolysis that, on the contrary, has taken place on the optical isomer (R) (II) and b) (R) alcohol, corresponding to the latter and having formula (III), after having been separated as mentioned

hereinbefore, are then hydrolyzed singly according to known techniques such as, for instance, alkaline hydrolysis etc., in order to obtain the respective hydrolysis products consisting of the 2-amino-1-alkanols having formula (I) in their corresponding (R) and (S) forms which finally are recovered, for instance by distillation etc., from the aqueous medium in their optically pure state.

The following examples merely serve to illustrate the invention and do not limit it in any way.

The following abbreviations have been used:

F.I.P.U = lipolytic activity expressed in F.I.P. units per mg.
EtOH = ethyl alcohol
e. e. = enantiomeric excess.

The identity of all the products having formulas (I), (II) and (III) was confirmed by N.M.R. analyses at 300 MHz.

The enantiomeric excesses relating to the 2-amino-1-alkanol isomers having formula (I) were determined by polarimetric data compared with the literature data.

## EXAMPLE 1

a) Preparation of racemic esters having formula (II):

A mixture containing 8.9 g of (R, S) 2-amino-1-butanol and 10.8 g of $Na_2CO_3$ in 40 ml of $H_2O$ was treated at O°C with 10.8 g of ethyl chloroformate. The whole was kept under stirring at room temperature for two hours, then the product was extracted with chloroform and the solution was dried over calcium chloride.
After evaporation of the solvent one obtained 14.8 g of (R, S) 2-amino-N-ethoxycarbonyl-1-butanol (yield 92%) that could be purified again at reduced pressure (b.p. 105-107°C at 67 Pa (0.5 mm Hg) [NMR, $\delta$ = 5.2 (d,1 H); 4.1 (q, 2 H); 3.4-3.7 (m, 4 H); 1.35-1.65 (m, 2 H); 1.25 (t, 3 H); 0.95 (t, 3 H) - in $CDCl_3$].
14 g of said product were dissolved in 40 ml of chloroform and 8.4 ml of pyridine. 6.8 ml of acetyl chloride were dropped, over 30 minutes, into the solution, cooled down to 0°C. The whole was kept under stirring at room temperature for 40 minutes and the mixture, thus obtained, was washed with a saturated solution of sodium bicarbonate (50 ml), then with an aqueous solution of 5% HCl (50 ml) and with $H_2O$ till neutrality was reached.
17 g of racemic 2-amino-N-ethoxycarbonyl-1-butanol acetate were obtained by evaporation of the solvent.
N.M.R. analysis (in $CDCl_3$) gave:
N.M.R., $\delta$ = 5.0 (d. 1H); 4.0-4.2 (m. 4H); 3.8 (m. 1H); 2.1 (s. 3H); 1.4-1.6 (m. 2H); 1.2 (t. 3H); 0.9 (t. 3H).
Starting from the other corresponding racemic 2-amino-1-alkanols having formula (I), alkyl chloroformates having formula (IV) and chlorides of acids R"-COOH, and by operating under the same conditions, the other racemic esters having formula (II) were obtained, as indicated in Table 1.

b) Enzymatic separation of the optical isomers of racemic esters having formula (II).

42 g of racemic 2-amino-N-ethoxycarbonyl-1-butanol acetate obtained in step a) were added to 300 ml of a buffer solution consisting of a 0.1 N solution of sodium and potassium phosphate at pH 7, containing 1 g of pancreatin (produced by the firm UNIBIOS at Trecate (Italy), having 57 F.I.P.U/mg).
Then the mixture was kept under stirring at 20°C over 18 hours, maintaining the pH at value 7 by addition of 7N aqueous NaOH. Then the mixture was extracted with toluene (3 x 300 ml).
17 g of S (-) 2-amino-N-ethoxycarbonyl-1-butanol acetate were obtained by evaporation of the solvent.
17 g of said product were treated with 150 ml of 30% aqueous NaOH over 3 hours at 60°C. Then the aqueous solution was distilled, thereby recovering 6.9 g of S (+) 2-amino-1-butanol having : $[\alpha]_D^{20}$ = + 12.4° (C=2, EtOH), e.e. = 99.2%.
Then the aqueous phase coming from the enzymatic hydrolysis was extracted again with $CHCl_3$ (3 x 300 ml).
By evaporation of the organic solvent 16 g of R(+) 2-amino-N-ethoxycabonyl-1-butanol (III) were obtained.
16 g of said product were treated with 70 ml of aqueous 30% NaOH, for 3 hours at 60°C. Then the aqueous solution was distilled, thereby recovering 8.9 g of R(-) 2-amino-1-butanol, $[\alpha]_D^{20}$ = -9.4°, (C=2,EtOH); e.e. = 75%.

## EXAMPLES 2-7

By using other racemic esters having formula (II), prepared starting from the corresponding racemic 2-amino-1-alkanols having formula (I), alkyl chloroformates having formula (IV) and acids having formula R″-

COOH and by operating under the same conditions as described in example 1 a) and 1 b), the optically pure 2-amino-1-alkanols indicated in the following Table 1 were obtained.
In examples 2, 5 and 7 the lipase used was Steapsin instead of pancreatin which was produced by the firm Sigma Chem. Co. St. Louis, USA, having a protein content of 35% and an activity equal to 30-70 units per mg of protein.

TABLE 1

| Example No. | R | R' | R" | amount of racemic ester (II) (g) | amount of enzyme (g) | | time (h) | (S) 2-amino-1-alkanol (I) g | $[\alpha]^{20}_{D}$ | e.e. % | (R) 2-amino-1-alkanol (I) g | $[\alpha]^{20}_{D}$ | e.e. % |
|---|---|---|---|---|---|---|---|---|---|---|---|---|---|
| 2 | $C_2H_5$ | $C_2H_5$ | $CH_3$ | 58 | Steapsin | 1.4 | 20 | 10.9 | + 12.3° | 98.4 | 10.4 | -12.4° | 99.2 |
| 3 | $C_2H_5$ | $C_2H_5$ | $CH_2CH_2CH_3$ | 56 | Pancreatin | 1.2 | 4 | 10.3 | + 11.2° | 89.6 | 8.4 | -12.4° | 99.2 |
| 4 | $C_2H_5$ | $nC_4H_9$ | $CH_3$ | 60 | Pancreatin | 1.2 | 27 | 10.1 | + 12.3° | 98.4 | 10.4 | -11.8° | 94.4 |
| 5 | $C_2H_5$ | $C_6H_5CH_2$ | $CH_3$ | 70 | Steapsin | 1.0 | 48 | 7.1 | + 12.4° | 99.2 | 13.6 | -9.1° | 72.8 |
| 6 | $CH_3$ | $C_2H_5$ | $CH_3$ | 48 | Pancreatin | 0.6 | 20 | 8.0 | + 22.2° | 96.5 | 8.6 | -21.9° | 95.2 |
| 7 | $CH_3$ | $C_2H_5$ | $CH_3$ | 48 | Steapsin | 1.3 | 14 | 9.0 | + 20.2° | 87.8 | 7.9 | -22.4° | 97.4 |

## Claims

1. Process for the biotechnological separation, carried out by means of enzymes, of the optical (R) and (S) isomers of racemic 2-amino-1-alkanols having general formula (I):

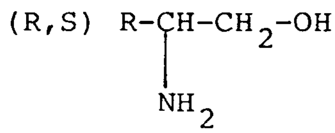

(I)

which process is characterized in that a racemic (R,S) N-alkoxycarbonyl ester derivative of said 2-amino-1-alkanols (I) having general formula (II):

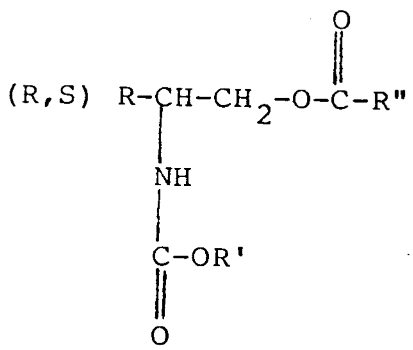

(II)

wherein R' represents a $C_1$-$C_8$ alkyl group or a benzyl group and R and R", which may be the same or different, represent $C_1$-$C_8$ alkyl groups, is reacted with a lipase which hydrolyzes, asymmetrically and in a prevalent manner, the (R) form of the racemic ester having formula (II) and in that, subsequently, by operating substantially according to known techniques, the obtained (R) alcohol, substantially corresponding to the (R) form of said hydrolyzed racemic ester (II), is separated from nonreacted ester (II), substantially in its (S) form, and then both the (R) alcohol and the non-reacted ester (II) in its (S) form are hydrolyzed separately to obtain the (R) and (S) 2-amino-1-alkanol compounds (I) which are optically pure.

2. Process according to claim 1, wherein the lipase is selected from pancreatin and steapsin.

3. Process according to claim 1 or 2, wherein the weight ratio of lipase to racemic ester of formula (II) ranges from 1:1 to 1:200.

4. Process according to claim 3, wherein the weight ratio of lipase to racemic ester of formula (II) ranges from 1:20 to 1:100.

5. Process according to any one of claims 1 to 4, wherein the enzymatic asymmetric hydrolysis is carried out at a temperature of from 5 to 60 ° C.

6. Process according to claim 5, wherein the enzymatic asymmetric hydrolysis is carried out at a temperature of from 20 to 40 ° C.

7. Process according to any one of claims 1 to 6, wherein the enzymatic asymmetric hydrolysis is carried out at a pH value ranging from 5 to 9, using a basic buffer solution or a mineral base.

8. Process according to any one of claims 1 to 7, wherein the lipase is immobilized on a substrate.

9. Process according to any one of claims 1 to 8, wherein the concentration of racemic ester (II) in the enzymatic asymmetric hydrolysis mixture is from 0.1 to 1 mole/ litre of mixture.

**10.** Process according to any one of claims 1 to 9, wherein the (S) N-alkoxycarbonyl ester derivative of 2-amino-1-alkanol (I) having formula (II) and the (R) alcohol obtained by enzymatic hydrolysis are isolated and hydrolyzed separately by alkaline hydrolysis.

## Revendications

**1.** Procédé pour la séparation biotechnologique effectuée au moyen d'enzymes, des isomères optiques (R) et (S) des 2-amino-1-alcanols racémiques ayant la formule générale (I):

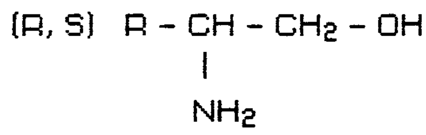

(I)

lequel procédé est caractérisé en ce qu'un ester racémique d'un dérivé (R, S) N-alcoxycarbonyle de ces 2-amino-1-alcanols racémiques(I) représenté par la formule générale (II) :

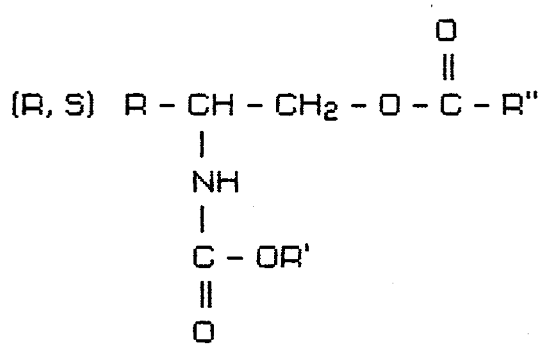

(II)

dans laquelle R' est un groupe alkyle en $C_{1-8}$ ou benzyle et R et R", qui peuvent être identiques ou différents, sont des groupes alkyles en $C_{1-8}$, est traité avec une lipase qui hydrolyse de façon asymétrique et prédominante la forme (R) de l'ester racémique ayant la formule (II) et en ce qu'ensuite, pratiquement selon des techniques usuelles, l'alcool (R) obtenu correspondant notablement à la forme (R) de l'ester racémique hydrolysé (II) est séparé de l'ester (II) n'ayant pas réagi notablement sous sa forme (S), et ensuite l'alcool (R) et l'ester (II) n'ayant pas réagi sous sa forme (S)> sont hydrolysés séparément pour donner les composés (R) et (S) 2-amino-1-alcanols (I) qui sont optiquement purs.

**2.** Procédé suivant la revendication 1 dans lequel la lipase est choisie parmi la pancréatine et la stéapsine.

**3.** Procédé suivant la revendication 1 ou 2, dans lequel le rapport pondéral de la lipase à l'ester racémique de formule (II) est de l'ordre de 1/1 à 1/200.

**4.** Procédé suivant la revendication 3, dans lequel le rapport pondéral de la lipase à l'ester racémique de formule (II) est de l'ordre de 1/20 à 1/100.

**5.** Procédé suivant l'une quelconque des revendications 1 à 4 dans lequel l'hydrolyse asymétrique enzymatique est effectuée à une température de 5 à 60 ° C.

**6.** Procédé suivant la revendication 5, dans lequel l'hydrolyse asymétrique enzymatique est effectuée à une température de 20 à 40 ° C.

**7.** Procédé suivant l'une quelconque des revendications 1 à 6 dans lequel l'hydrolyse asymétrique enzymatique est effectuée à un pH de l'ordre de 5 à 9 au moyen d'une solution tampon basique ou d'une base minérale.

**8.** Procédé suivant l'une quelconque des revendications 1 à 7, dans lequel la lipase est immobilisée sur

un substrat.

**9.** Procédé suivant l'une quelconque des revendications 1 à 8 dans lequel la concentration de l'ester racémique (II) dans le mélange d'hydrolyse asymétrique enzymatique est de 0,1 à 1 mole/litre de mélange.

**10.** Procédé suivant l'une quelconque des revendications 1 à 9 dans lequel l'ester de formule (II) du dérivé (S)-N-alcoxycarbonyle du 2-amino-1-alcanol (I) et l'alcool (R) obtenu par hydrolyse enzymatique, sont isolés et hydrolysés séparément par hydrolyse alcaline.

**Patentansprüche**

**1.** Verfahren zur biotechnischen Trennung der optischen (R)- und (S)-Isomeren von racemischen 2-Amino-1-alkanolen der allgemeinen Formel (I):

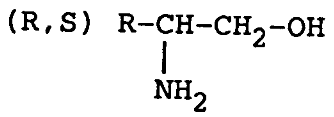

(I)

unter Verwendung von Enzymen, welches Verfahren dadurch gekennzeichnet ist, daß ein racemisches (R,S)-N-Alkoxycarbonylester-Derivat des 2-Amino-1-alkanols (I) der allgemeinen Formel (11):

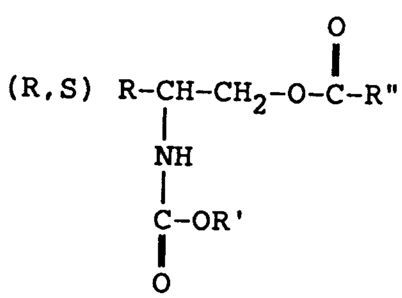

(II)

worin R' eine $C_1$-$C_8$ Alkylgruppe oder eine Benzylgruppe darstellt, und R und R", gleich oder verschieden voneinander, $C_1$-$C_8$ Alkylgruppen bedeuten, mit einer Lipase umgesetzt wird, die asymmetrisch und in bevorzugter Weise die (R)-Form des racemischen Esters der Formel (II) hydrolysiert, und dadurch, daß anschließend im wesentlichen nach bekannten Verfahren der erhaltene (R)-Alkohol, der im wesentlichen der (R)-Form des hydrolysierten racemischen Esters (II) entspricht, von nicht-umgesetzem Ester (II), der im wesentlichen in seiner (S)-Form vorliegt, abgetrennt wird, und dann sowohl der (R)-Alkohol als auch der nicht-umgesetzte Ester (11) in seiner (S)-Form separat hydrolysiert werden, um die (R)- und (S)-2-Amino-1-alkanol-Verbindungen (I) zu erhalten, die optisch rein sind.

**2.** Verfahren nach Anspruch 1, worin die Lipase aus Pankreatin und Steapsin ausgewählt ist.

**3.** Verfahren nach Anspruch 1 oder 2, worin das Gewichtsverhältnis von Lipase zu dem racemischen Ester der Formel (II) 1:1 bis 1:200 beträgt.

**4.** Verfahren nach Anspruch 3, worin das Gewichtsverhältnis von Lipase zu dem racemischen Ester der Formel (II) 1:20 bis 1:100 beträgt.

**5.** Verfahren nach irgendeinem der Ansprüche 1 bis 4, worin die enzymatische asymmetrische Hydrolyse bei einer Temperatur von 5 bis 60 ° C durchgeführt wird.

6. Verfahren nach Anspruch 5, worin die enzymatische asymmetrische Hydrolyse bei einer Temperatur von 20 bis 40 ° C durchgeführt wird.

7. Verfahren nach irgendeinem der Ansprüche 1 bis 6, worin die enzymatische asymmetrische Hydrolyse bei einem pH-Wert von 5 bis 9 unter Verwendung einer basischen Pufferlösung oder einer Mineralbase durchgeführt wird.

8. Verfahren nach irgendeinem der Ansprüche 1 bis 7, worin die Lipase auf einem Träger immobilisiert ist.

9. Verfahren nach irgendeinem der Ansprüche 1 bis 8, worin die Konzentration des racemischen Esters (II) in der Mischung für die enzymatische asymmetrische Hydrolyse 0,1 bis 1 Mol pro Liter der Mischung beträgt.

10. Verfahren nach irgendeinem der Ansprüche 1 bis 9, worin das (S)-N-Alkoxycarbonylester-Derivat des -2-Amino-1-alkanols (I) der Formel (II) und der durch enzymatische Hydrolyse erhaltene (R)-Alkohol isoliert und separat durch alkalische Hydrolyse hydrolysiert werden.